(19) Europäisches Patentamt
European Patent Office
Office européen des brevets

(11) Veröffentlichungsnummer: **0 065 189**
**B1**

(12) # EUROPÄISCHE PATENTSCHRIFT

(45) Veröffentlichungstag der Patentschrift: **24.09.86**

(21) Anmeldenummer: **82103833.8**

(22) Anmeldetag: **05.05.82**

(51) Int. Cl.[4]: **C 07 D 233/38**, C 07 D 235/02, C 07 D 241/08, C 07 D 241/38, C 07 D 241/44, A 01 N 25/32

(54) **Heterocyclische Dihalogenacetamide, Verfahren zu ihrer Herstellung und herbizide Mittel, die Acetanilide als herbizide Wirkstoffe und diese Dihalogenacetamide als antagonistische Mittel enthalten.**

(30) Priorität: **14.05.81 DE 3119077**

(43) Veröffentlichungstag der Anmeldung: **24.11.82 Patentblatt 82/47**

(45) Bekanntmachung des Hinweises auf die Patenterteilung: **24.09.86 Patentblatt 86/39**

(84) Benannte Vertragsstaaten: **AT BE DE FR GB IT NL**

(56) Entgegenhaltungen:
**EP-A-0 007 588**
**EP-A-0 023 287**
**EP-A-0 023 305**
**US-A-4 240 961**

(73) Patentinhaber: **BASF Aktiengesellschaft, Carl-Bosch-Strasse 38, D-6700 Ludwigshafen (DE)**

(72) Erfinder: **Plath, Peter, Dr., Berner Weg 24, D-6700 Ludwigshafen (DE)**
Erfinder: **Rohr, Wolfgang, Dr., In der Dreispitz 13, D-6706 Wachenheim (DE)**
Erfinder: **Schwendemann, Volker, Dr., Hauptstrasse 64 A, D-6901 Wiesenbach (DE)**
Erfinder: **Wuerzer, Bruno, Dr. Dipl.- Landwirt, Ruedigerstrasse 13, D-6701 Otterstadt (DE)**

EP 0 065 189 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft heterocyclische Dihalogenacetamide, Verfahren zu ihrer Herstellung und herbizide Mittel, die Acetanilide als herbizide Wirkstoffe und diese Dihalogenacetamide als antagonistische Mittel enthalten.

Acetanilide der Formel

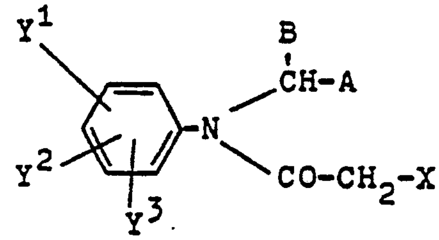

(II),

in der

$Y^1$ Wasserstoff, einen unverzweigten oder verzweigten Alkyl- oder Alkoxyrest mit bis zu 5 Kohlenstoffatomen,

$Y^2$ und $Y^3$ Wasserstoff, Halogen, einen unverzweigten oder verzweigten Alkyl- oder Alkoxyrest mit bis zu 5 Kohlenstoffatomen,

$Y^1$ zusammen mit $Y^2$ eine orthoständig verknüpfte, gegebenenfalls durch unverzweigte oder verzweigte Alkylgruppen mit bis zu 4 Kohlenstoffatomen substituierte Alkylenketten mit bis zu 6 Kohlenstoffatomen,

X Chlor oder Brom,

A einen Alkoxy- oder Alkoxyalkylrest mit bis zu 4 Kohlenstoffatomen oder einen Azolylrest, der einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, wobei A auch für Salze der Azolylreste stehen kann, die 2 oder 3 Stickstoffatome enthalten, und

B Wasserstoff oder Methyl bedeuten, sind herbizid ausgezeichnet wirksam, führen aber bei Anwendung in Kulturen, wie Mais, oder auch in anderen Kulturen aus der Familie der Gramineen zu Schädigungen der Nutzpflanzen.

Aufgabe der Erfindung war es deshalb, antagonistische Mittel zu finden, die diese Unverträglichkeit der herbiziden Acetanilide gegenüber bestimmten Kulturpflanzen kompensieren.

Herbizide Mittel, die neben herbiziden Wirkstoffen Dichloracetamide als antagonistisch wirkende Verbindungen enthalten, sind aus der DE-OS 22 18 097 und der DE-OS 22 45 471 bekannt. Die in der DE-OS 22 18 097 beschriebenen Dichloracetamide werden vorwiegend zur Antagonisierung unerwünschter Kulturpflanzenschädigungen durch Thiolcarbamate verwendet, während aus der DE-OS 22 45 471 auch herbizide Mittel bekannt sind, die Dichloracetamide und Chloracetanilide, beispielsweise 2-Chlor-2',6'-diethyl-N-butoxymethyl-acetanilid oder 2-Chlor-2',6'-diethyl-N-methoxymethyl-acetanilid, enthalten.

Es wurde gefunden, daß heterocyclische Dihalogenacetamide der Formel

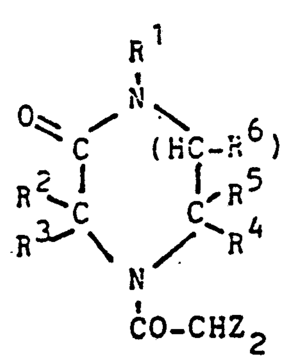

(I),

in der

$R^1$ Wasserstoff oder Alkyl mit 1 bis 4 Kohlenstoffatomen,

$R^2$ Wasserstoff oder Methyl,
$R^3$ Wasserstoff oder Alkyl mit 1 bis 3 Kohlenstoffatomen,
$R^4$ Wasserstoff, Alkyl mit 1 bis 4 Kohlenstoffatomen oder Cyclohexyl,
$R^5$ Wasserstoff oder Methyl,
$R^6$ Wasserstoff oder Methyl,
Z Chlor oder Brom und
n 0 oder 1 bedeuten, oder in der $R^2$ zusammen mit $R^3$ einen Pentamethylenrest bildet, oder in der
$R^4$ zusammen mit $R^5$ einen Tetra- oder Pentamethylenrest bildet, wenn
n 0 ist, oder in der
$R^5$ zusammen mit $R^6$ einen Tetramethylenrest bildet, wenn
n 1 ist, mit der Maßgabe, daß $R^1$, $R^2$, $R^3$, $R^4$, $R^5$ und $R^6$
nicht gleichzeitig Wasserstoff bedeuteten, wenn n 1 ist, sich ausgezeichnet zur Steigerung der Kulturpflanzenverträglichkeit von herbiziden Acetaniliden der Formel II eignen. Herbizide Mittel, die ein Acetanilid der Formel II und ein heterocyclisches Dihalogenacetamid der Formel I enthalten, können sowohl in Mais als auch in anderen Getreidekulturen eingesetzt werden. Dabei bleibt die gute herbizide Wirkung der Acetanilide erhalten, während Schädigungen an den Nutzpflanzen ganz oder weitgehend unterbunden werden.

Als antagonistische Mittel kommen heterocyclische Dihalogenacetamide der Formel I in Betracht, bei denen n 0 oder 1 bedeutet, so daß es sich sowohl um 1-Dihalogenacetyl-imidazolidin-4-one als auch um 1-Dihalogenacetyl-piperazin-3-one handeln kann. In Formel I stehen $R^1$ für Wasserstoff oder für Alkylreste mit 1 bis 4 Kohlenstoffatomen, wie Methyl, Ethyl, Isopropyl, Isobutyl, $R^2$ für Wasserstoff oder Methyl, $R^3$ für Wasserstoff oder für Alkylreste mit 1 bis 3 Kohlenstoffatomen, wie Methyl, Ethyl, Isopropyl, $R^4$ für Wasserstoff Alkylreste mit 1 bis 4 Kohlenstoffatomen, wie Methyl, Ethyl, Isopropyl, tert.-Butyl oder für Cyclohexyl und $R^5$ und $R^6$ jeweils für Wasserstoff oder Methyl. $R^2$ kann auch zusammen mit $R^3$ einen Pentamethylenrest bilden. Außerdem kann $R^4$ zusammen mit $R^5$ einen Tetra- oder Pentamethylenrest bilden, wenn n 0 ist oder $R^5$ kann zusammen mit $R^6$ einen Tetramethylenrest bilden, wenn n 1 ist. Für den Fall, daß n 1 ist, sollen $R^1$, $R^2$, $R^3$, $R^4$, $R^5$ und $R^6$ aber nicht gleichzeitig Wasserstoff bedeuten.

Beispiele für antagonistisch wirkende Dihalogenacetamide der Formel I sind 1-Dichloracetyl-2-spirocyclohexyl-4-oxo-imidazolidin, 1-Dichloracetyl-2-isopropyl-4-oxo-imidazolidin, 1-Dichloracetyl-2,5-dimethyl-2-isopropyl-4-oxo-imidazolidin, 1-Dichloracetyl-2-isopropyl-4-oxo-5-methyl-imidazolidin, 1-Dichloracetyl-2-spirocyclopentyl-4-oxo-imidazolidin, 1-Dichloracetyl-2,3-diisopropyl-4-oxo-imidazolidin, 1-Dichloracetyl-2-isopropyl-4-oxo-5,5-dimethyl-imidazolidin, 1-Dichloracetyl-2-tert.-butyl-4-oxo-imidazolidin, 1-Dichloracetyl-2-cyclohexyl-4-oxo-imidazolidin, 1-Dichloracetyl-2-isopropyl-3-methyl-4-oxo-imidazolidin, 1-Dichloracetyl-2-tert.-butyl-3-methyl-4-oxo-imidazolidin, 1-Dichloracetyl-2-cyclohexyl-4-oxo-imidazolidin, 1-Dichloracetyl-2-cyclohexyl-3-methyl-4-oxo-imidazolidin, 1-Dibromacetyl-2-tert.-butyl-3-methyl-4-oxo-imidazolidin, 1-Dibromacetyl-2-cyclohexyl-4-oxo-imidazolidin, 1-Dichloracetyl-2-methyl-3-oxo-piperazin, 1-Dichloracetyl-2,5-dimethyl-3-oxo-piperazin, 1-Dichloracetyl-2,2-dimethyl-3-oxo-piperazin, 1-Dichloracetyl-2,5,6-trimethyl-3-oxo-piperazin, 1-Dichloracetyl-2-methyl-3-oxo-5,6-tetramethylen-piperazin, 1-Dichloracetyl-2-ethyl-3-oxo-piperazin, 1-Dichloracetyl-2-isopropyl-3-oxo-piperazin, 1-Dichloracetyl-2-spirocyclohexyl-2-oxo-piperazin, 1-Dichloracetyl-2-spirocyclohexyl-3-oxo-5,6-tetramethylen-piperazin, 1-Dibromacetyl-2,2-dimethyl-3-oxo-piperazin, 1-Dibromacetyl-2-methyl-3-oxo-piperazin.

Heterocyclische Dichloracetamide der Formel I können durch Umsetzung von Aminen der Formel

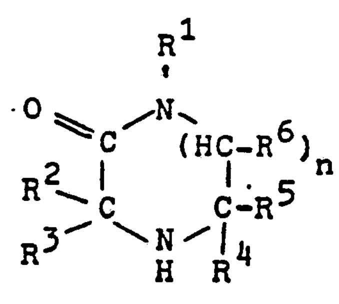

(III),

in der
$R^1$ bis $R^6$ und n die oben genannten Bedeutungen haben, mit Dihalogenacetylchloriden der Formel $Z_2CH$-COCl, in der Z Chlor oder Brom bedeutet, in Gegenwart eines halogenwasserstoffbindenden Mittels und eines Lösungsmittels erhalten werden. Die Umsetzung wird bei einer Temperatur im Bereich zwischen 0 und 30°C durchgeführt.

Als Lösungsmittel eignen sich wasserfreie Solventien, z.B. gegebenenfalls halogenierte Kohlenwasserstoffe, wie Toluol, Xylole, Chlorbenzol, Dichlormethan, Ethylenchlorid, Ether, wie Diethylether, Methyl-tert.-butylether, Tetrahydrofuran, 1,4-Dioxan, oder Nitrile, wie Acetonitril, oder zweiphasige Systeme, z.B. Wasser

und Toluol, Wasser und Dichlormethan.

Geeignete halogenwasserstoffbindende Mittel sind Alkalimetallcarbonate, Alkalimetallhydrogencarbonate, wäßrige Lösungen von Alkalimethallhydroxiden, Trialkylamine, N,N-Dialkylaniline, wie N,N-Dimethylanilin und Pyridinbasen. Das Amin der Formel III wird zweckmäßigerweise mit der äquimolaren Menge oder der 1,2-fachen molaren Menge an Dihalogenacetylchlorid ungesetzt. Pro Mol Dihalogenacetylchlorid werden 1 bis 1,2 Mol halogenwasserstoffbindendes Mittel zugegeben.

Zur Herstellung von Imidazolidin-4-onen der Formel III setzt man Ketone oder Aldehyde mit Nitrilen oder Amiden von α-Aminosäuren um (J. Chem. Soc. 1951, 3479). Diese Verbindungen lassen sich auch durch Umwandlung von α-Aminosäureestern in Schiff'sche Basen und anschließende Umsetzung mit Ammoniak oder primären Aminen erhalten (Rec. Trav. Chim. 111, 284 (1971)).

Piperazinone der Formel III werden z.B. durch Umsetzung von Cyanhydrinen mit aliphatischen 1,2-Diaminen erhalten (US-PS 2 700 668).

**Herstellung der Amine der Formel III**

1. Zu einer Suspension von 77 g (0,69 Mol) Glycinamidhydrochlorid in 300 ml Toluol gibt man 137 g Cyclohexanon und tropft anschließend eine Mischung aus 120 ml Methanol und 125,5 g (0,7 Mol) einer 30 %igen $NaOCH_3$-Lösung bei 25°C zu. Nach 4 Stunden Kochen unter Rückfluß kühlt man auf 5°C ab, saugt das abgeschiedene NaCl ab und dampft das Filtrat ein. Nach Anreiben des Rückstandes mit Ligroin erhält man 44 g (41 % d.Th.) 2-Spiro-cyclohexyl-4-oxo-imidazolidin vom Fp. 118-119°C.

2. 279 g (2 Mol) Alaninmethylesterhydrochlorid werden in 100 ml Wasser gelöst und rasch mit 216 g (3 Mol) Isobutyraldehyd versetzt. Danach tropft man bei 10 bis 15°C 242 g (2,4 Mol) Triethylamin zu und läßt 16 Stunden lang nachrühren. Nach Extraktion der Reaktionsmischung mit 250 ml Methyl-tert.-butylether wird die organische Phase getrocknet und unter vermindertem Druck eingeengt. Man erhält 281 g (89 % d.Th.) eines Öls der Formel

$(CH_3)_2CH\text{-}CH=N\text{-}CH(CH_3)\text{-}COOCH_3$.

62,8 g (0,4 Mol) dieser Schiff'schen Base werden in 200 ml Methanol gelöst und bei 25°C bis zur Sättigung mit gasförmigem Methylamin versetzt. Nach 16 Stunden Nachrühren wird unter vermindertem Druck eingeengt. Man erhält 58 g (93 % 3,5-Dimethyl-2-isopropyl-4-oxo-imidazolidin (Öl).

3. Analog der unter 2. beschriebenen Umsetzung erhält man aus Glycinmethylesterhydrochlorid und Isobutyraldehyd in 90 %iger Ausbeute N-(2-Methyl-propyliden)-glycinethylester vom Kp 53-56°C/0,1 mbar. Die Umsetzung dieser Schiff'schen Base mit gasförmigem Ammoniak in Methan ergibt in 80 %iger Ausbeute 2-Isopropyl-4-oxo-imidazolidin (Öl).

4. Zu einer Lösung von 176 g (2 Mol) 2,3-Diaminobutan in 250 ml Wasser werden unter Kühlung tropfenweise 138,5 g (1,95 Mol) Acetaldehydcyanhydrin gegeben, wobei die Temperatur bei 25°C gehalten wird. Danach wird zum Sieden erhitzt, bis nach 8 Stunden die Ammoniak-Abspaltung beendet ist. Das Reaktionsgemisch wird anschließend unter vermindertem Druck destilliert. Man erhält 200 g (72 % d.Th.) 2,5,6-Timethyl-3-oxo-piperazin vom Kp 123°C/0,5 mbar.

5. Analog der unter 4. beschriebenen Umsetzung erhält man aus 1,2-Diaminocyclohexan und Acetaldehydcyanhydrin 2-Methyl-3-oxo-5,6-tetramethylen-piperazin vom Fp. 177-178°C.

6. Analog der unter 4. beschriebenen Umsetzung erhält man aus Ethylendiamin und Acetoncyanhydrin 2,2-Dimethyl-3-oxo-piperazin vom Fp. 133-135°C.

**Herstellung der heterocyclischen Dihalogenacetamide der Formel I**

I. Man löst 45 g (0,35 Mol) 2-Isopropyl-4-oxo-imidazolidin (Beispiel 3) in 250 ml Methyl-tert.-butylether, versetzt mit 17,2 g (0,43 Mol) Natriumhydroxid in 20 ml Wasser und tropft anschließend 57 g (0,39 Mol) Dichloracetylchlorid bei 10 bis 15°C zu. Nach 10-stündigem Rühren saugt man den ausgefallenen Feststoff ab, wäscht mit Wasser nach, und trocknet unter vermindertem Druck. Man erhält 41 g (49 %. d.Th.) 1-Dichloracetyl-2-isopropyl-4-oxo-imidazolidin vom Fp. 172-173°C.

II. Zu einer Lösung von 20 g (0,18 Mol) 2-Methyl-piperazinon-(3) in 150 ml Methylenchlorid wird eine Lösung von 8 g (0,2 Mol) Natriumhydroxid in 15 ml Wasser gegeben. Danach werden bei 10 bis 15°C 26,6 g (0,18 Mol) Dichloracetylchlorid zugetropft. Nach 10-stündigem Rühren bei 25°C wird vom ausfallenden Rohprodukt abgesaugt. Anschließend wird mit Wasser nachgewaschen und aus Isopropanol/$H_2O$ (2:1) umkristallisiert. Man erhält 14,4 g (36 % d.Th.) 1-Dichloracetyl-2-methyl-3-oxo-piperazin vom Fp. 167-168°C.

Entsprechend werden die in den Tabellen 1 und 2 aufgeführten Dihalogenacetamide erhalten.

**Tabelle 1**

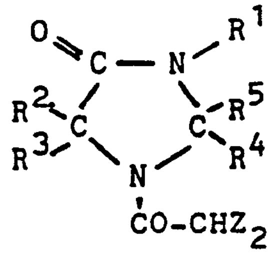

| Nr. | $R^1$ | $R^2$ | $R^3$ | $R^4$ | $R^5$ | Z | Fp [°C] |
|---|---|---|---|---|---|---|---|
| 1 | H | H | H | $i\text{-}C_3H_7$ | H | Cl | 172-173 |
| 2 | H | H | $CH_3$ | $i\text{-}C_3H_7$ | H | Cl | 198-199 |
| 3 | $CH_3$ | H | $CH_3$ | $i\text{-}C_3H_7$ | H | Cl | 114-115 |
| 4 | H | H | H | $-(CH_2)_5-$ | | Cl | 218 |
| 5 | $i\text{-}C_3H_7$ | H | H | $i\text{-}C_3H_7$ | H | Cl | |
| 6 | H | $CH_3$ | $CH_3$ | $i\text{-}C_3H_7$ | H | Cl | |
| 7 | H | H | H | tert.-$C_4H_9$ | H | Cl | 174-176 |
| 8 | H | H | H | $C_6H_{11}$ | H | Cl | |
| 9 | $CH_3$ | H | H | $i\text{-}C_3H_7$ | H | Cl | |
| 10 | $CH_3$ | H | H | tert.-$C_4H_9$ | H | Cl | 112 |
| 11 | H | H | H | $C_6H_{11}$ | H | Cl | 209 |
| 12 | $CH_3$ | H | H | $C_6H_{11}$ | H | Cl | 152 |
| 13 | H | H | H | $C_6H_{11}$ | H | Br | 202 |
| 14 | $CH_3$ | H | H | $C_6H_{11}$ | H | Br | |
| 15 | $n\text{-}C_4H_9$ | H | H | tert.-$C_4H_9$ | H | Cl | |
| 16 | $i\text{-}C_3H_7$ | H | H | tert.-$C_4H_9$ | H | Cl | |
| 17 | H | $CH_3$ | $CH_3$ | $i\text{-}C_3H_7$ | H | Cl | |
| 18 | $CH_3$ | $CH_3$ | $CH_3$ | $i\text{-}C_3H_7$ | H | Cl | |
| 19 | $CH_3$ | H | H | tert.-$C_4H_9$ | H | Br | 141 |

**Tabelle 2**

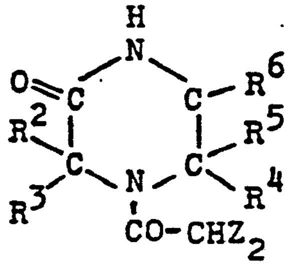

| Nr. | $R^2$ | $R^3$ | $R^4$ | $R^5$ | $R^6$ | Z | Fp [°C] |
|---|---|---|---|---|---|---|---|
| 21 | H | $CH_3$ | H | H | H | Cl | 167–168 |
| 22 | $CH_3$ | H | H | H | $CH_3$ | Cl | 153–155 |
| 23 | $CH_3$ | $CH_3$ | H | H | H | Cl | 196–197 |
| 24 | H | $CH_3$ | $CH_3$ | H | $CH_3$ | Cl | 145–147 |
| 25 | $CH_3$ | H | H | $-(CH_2)_4-$ | | Cl | 247–248 |
| 26 | H | $C_2H_5$ | H | H | H | Cl | |
| 27 | H | i-$C_3H_7$ | H | H | H | Cl | |
| 28 | $-(CH_2)_5-$ | | H | H | H | Cl | |
| 29 | $-(CH_2)_5-$ | | H | $-(CH_2)_4$ | | Cl | |
| 30 | H | $CH_3$ | H | H | H | Br | |
| 31 | H | $CH_3$ | $CH_3$ | H | H | Br | |
| 32 | $CH_3$ | $CH_3$ | H | H | H | Br | 135 (Zers.) |

Acetanilide, deren Kulturpflanzenverträglichkeit durch die Dihalogenacetamide der Formel I verbessert werden kann, sind solche der Formel II, bei denen $Y^1$ für Wasserstoff, Alkyl bis zu 5 Kohlenstoffatomen wie Methyl, Ethyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Ethoxy, Propoxy, Butoxy, Pentyloxy;

$Y^2$ und $Y^3$ für Wasserstoff, Halogen, wie Fluor, Chlor, Brom oder Jod, Alkyl bis zu 5 Kohlenstoffatomen, wie Methyl, Ethyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Ethoxy, Propoxy, Butoxy, Pentyloxy;

$Y^1$ zusammen mit $Y^2$ für eine orthoständig verknüpfte, gegegebenenfalls durch Alkyl mit bis zu 4 Kohlenstoffatomen substituierte Alkylenkette mit bis zu 6 Kohlenstoffatomen, wie Ethylen, Trimethylen, Tetramethylen, 1-Methyl-trimethylen, 1,1-Dimethyl-trimethylen, 1,1-Dimethyl-tetramethylen;

X für Chlor oder Brom, vorzugsweise Chlor,

A für einen Azolylrest, wie einen Pyrrolyl-, Pyrazolyl-, Imidazolyl-, 1,2,4-Triazolyl-, 1,2,3-Triazolyl-, Tetrazolylrest, der einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, oder für einen Alkoxy- oder Alkoxyalkylrest mit bis zu 4 Kohlenstoffatomen, wie Methoxy, Ethoxy, n-Butoxy, Methoxymethyl, 2-Methoxyethyl, Ethoxymethyl, und

B für Wasserstoff oder Methyl stehen.

Darüber hinaus kann der Rest A für den Fall, daß der Azolylrest 2 oder 3 Stickstoffatome enthält, auch salzartig an eine der üblichen starken anorganischen oder organischen Säuren, wie Chlorwasserstoffsäure, Salpetersäure, Schwefelsäure, Trichloressigsäure, Methansulfonsäure, Perfluorhexansulfonsäure oder Dodecylbenzolsulsonsäure gebunden sein.

Bevorzugt sind Acetanilide der Formel II, die in 2- und 6-Stellung am Phenylring Methyl oder Ethyl und in 3-Stellung Wasserstoff oder Methyl tragen. X steht vorzugsweise für Chlor, während für A insbesondere ein Azolylrest, wie Pyrazolyl, 3,5-Dimethylpyrazolyl, 2-Methyl-pyrazol-5-yl, 1,2,4-Triazolyl oder 4,5-Dichlorimidazol, Ethoxy, n-Butoxy oder Methoxymethyl und für B Wasserstoff oder Methyl in Betracht kommen.

Insbesondere enthalten die erfindungsgemäßen herbiziden Mittel folgende Acetanilide:

2-Chlor-2',6-dimethyl-N-(pyrazol-1-yl-methyl)-acetanilid, 2-Chlor-2'-methyl-6'-ethyl-N-(pyrazol-1-yl-methyl)-acetanilid, 22-Chlor-2',6'-dimethyl-N-(3,5-dimethylpyrazol-1-yl-methyl)-acetanilid, 2-Chlor-2',6'-dimethyl-N-(1,2,4-triazol-1-yl-methyl)-acetanilid, 2-Chlor-2',3',6'-trimethyl-N-(pyrazol-1-yl-methyl)-acetanilid, 2-Chlor-2'-methyl-6'-ethyl-N-(3,5-dimethyl-pyrazol-1-yl-methyl)-acetanilid, 2-Chlor-2',6'-diethyl-N-(3,5-dimethyl-pyrazol-1-yl-methyl)-acetanilid, 2-Chlor-2',3',6'-trimethyl-N-(3,5-dimethyl-pyrazol-1-yl-methyl)-acetanilid, 2-Chlor-2',6'-diethyl-N-(pyrazol-1-yl-methyl)-acetanilid, 2-Chlor-2',6'-dimethyl-N-(4,5-dichlorimidazol-1-yl-methyl)-acetanilid, 2-Chlor-2'-methyl-6'-ethyl-N-(4,5-dichlorimidazol-1-yl-methyl)-acetanilid, 2-Chlor-2',6'-diethyl-N-(4,5-dichlorimidazol-1-yl-methyl)-acetanilid, 2-Chlor-2'-methyl-6'-ethyl-N-(1,2,4-triazol-1-yl-methyl)-acetanilid, 2-Chlor-2',6'-diethyl-N-(1,2,4-triazol-1-yl-methyl)-acet-anilid, 2-Chlor-2',3',6'-trimethyl-N-(1,2,4-triazol-1-yl-methyl)-acetanilid, 2-Chlor-2',6'-dimethyl-N-(2-methoxy-ethyl)-acetanilid, 2-Chlor-2'-methyl-6'-ethyl-N-ethoxymethyl-acetanilid, 2-Chlor-2',6'-diethyl-N-(n-butoxymethyl)-acetanilid, 2-Chlor-2'-ethyl-6'-methyl-N-(1-methyl-2-methoxy-ethyl)-acetanilid und 2-Chlor-2',6'-dimethyl-N-[(2-methyl-pyrazol-5-yl)-methyl]-acetanilid.

Die Acetanilide der Formel II und ihre Herstellung sind Gegenstand der DE-OS 26 48 008, der DE-OS 27 44 396, der DE-OS 23 05 495, der DE-OS 23 28 340, der DE-OS 28 42 315 und der US-PS 3 547 620.

Herbizide Wirkstoffe und antagonistisch wirkende Verbindungen können gemeinsam oder getrennt vor oder nach der Saat in den Boden eingearbeitet werden. Bei den Acetaniliden ist das gebräuchlichste Anwendungsverfahren die Ausbringung auf die Erdoberfläche unmittelbar nach Ablage der Samen oder in der Zeit zwischen Einsaat und Auflaufen der jungen Pflanzen. Auch eine Behandlung während des Auflaufens ist möglich. In jedem Fall kann das antagonistisch wirkende Mittel gleichzeitig mit dem herbiziden Wirkstoff ausgebracht werden. Auch eine getrennte Ausbringung, wobei der Antagonist zuerst und anschließend der herbizide Wirkstoff oder umgekehrt auf das Feld gebracht werden, ist möglich, sofern zwischen Ausbringung beider Substanzen nicht soviel Zeit verstreicht, daß der herbizide Wirkstoff bereits Schaden an den Kulturpflanzen anrichtet. Wirkstoff und Antagonist können hierbei als Spritzmittel in suspendierbarer, emulgierbarer oder löslicher Form oder als Granulate getrennt oder gemeinsam formuliert vorliegen. Denkbar ist auch eine Behandlung der Kulturpflanzensamen mit dem Antagonisten vor der Aussaat. Der herbizide Wirkstoff wird dann allein in der üblichen Weise appliziert.

Für das gleiche herbizide Acetanilid werden unterschiedliche Menge einer antagonistisch wirkenden Verbindungen benötigt, wenn das Acetanilid in verschiedenen Kulturen eingesetzt wird. Die Mengenverhältnisse, in denen Acetanilid und Dihalogenacetamid eingesetzt werden, sind in breiten Bereichen variabel. Sie sind abhängig von der Struktur des Acetanilids, des Dihalogenacetamids und der jeweiligen Kultur. Geeignete Anteilsverhältnisse von herbizidem Wirkstoff : antagonistisch wirkender Verbindung liegen zwischen 1:2 bis 1:0,01, vorzugsweise 1:0,25 bis 1:0,05 Gewichtsteilen.

Die neuen herbiziden Mittel können neben Acetanilid und Dihalogenacetamid weitere herbizide oder wachstumsregulierende Wirkstoffe anderer chemischer Struktur enthalten, wobei der antagonistische Effekt erhalten bleibt. So können sie beispielsweise 2-Chlor-4-ethylamino-6-isopropylamino-1,3,5-triazin, 2-(2-Chlor-4-ethylamino-1,3,5-triazin-6-yl-amino)-2-methyl-propionitril, N-(1-Ethyl-n-propyl)-2,6-dinitro-3,4-dimethylanilin oder 1-(4-Isopropyl-phenyl)-3,3-dimethyl-harnstoff als zusätzliche herbizide Wirkstoffe enthalten.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenyl, Alkylphenolpolyglykolether, Tributylphenylpolyglykolether, Alkalarylpolyetheralkohole, Isotridecylalkohol,

Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubmittel können durch Mischen oder gemeinsames Vermahlen von verbizidem Wirkstoff und/oder Antidot mit einem festen Trägerstoff hergestellt werden. Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent an herbizidem Wirkstoff und Antidot, vorzugsweise zwischen 0,5 und 90 Gewichtsprozent. Die Aufwandmengen an herbizidem Wirkstoff betragen 0,2 bis 5 kg/ha. Diese Menge an herbizidem Wirkstoff wird, gemeinsam oder getrennt, mit einer solchen Menge an Antidot ausgebracht, daß das Anteilsverhältnis herbizider Wirkstoff : antagonistischer Verbindung 1:2 bis 1:0,01, vorzugsweise 1:0,25 bis 1:0,05, Gewichtsteile beträgt.

**Beispiele für Formulierungen sind:**

I. 40 Gewichtsteile der Mischung aus vier Gewichtsteilen 2-Chlor-2',6'-dimethyl-N-(pyrazol-1-yl-methyl)-acetanilid und einem Gewichtsteil 1-Dichloracetyl-2-isopropyl-4-oxo-imidazolidin werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

II. 3 Gewichtsteile der Mischung aus einem Gewichtsteil 2-Chlor-2'-methyl-6'-ethyl-N-(pyrazol-1-yl-methyl)-acetanilid und einem Gewichtsteile 1-Dichloracetyl-2,2-dimethyl-3-oxo-piperazin werden mit 97 Gewichtsteilen feinteiligem Kaolin innig vermischt. Man erhält auf diese Weise ein Stäubemittel, das 3 Gewichtsprozent des Wirkstoffs enthält.

III. 30 Gewichtsteile der Mischung aus einem Gewichtsteil 2-Chlor-2'-methyl-6'-ethyl-N-(1,2,4-triazol-1-yl-methyl)-acetanilid und zwei Gewichtsteilen 1-Dichloracetyl-2,5,6-trimethyl-3-oxo-piperazin werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Teile der Mischung aus 8 Gewichtsteilen 2-Chlor-2'-methyl-6'-ethyl-N-ethoxymethyl-acetanilid und einem Gewichtsteil 1-Dichloracetyl-2-isopropyl-3,5-dimethyl-4-oxo-imidazolidin werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

V. 20 Gewichtsteile der Mischung aus 10 Gewichtsteilen 2-Chlor-2',6'-dimethyl-N-(2-methoxyethyl)-acetanilid und einem Gewichtsteil 1-Dichloracetyl-2-methyl-3-oxo-5,6-tetramethylen-piperazin werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Der Einfluß verschiedener Vertreter der erfindungsgemäßen herbiziden Mittel auf das Wachstum von erwünschten und unerwünschten Pflanzen im Vergleich zu dem herbizider Mittel aus den gleichen herbiziden Wirkstoffen und einer antagonistisch wirkenden, bereits bekannten Verbindung chemisch ähnlicher Struktur wird durch die folgenden biologischen Beispiele belegt. Die Versuche zeigen, daß die Verträglichkeit der herbiziden Acetanilide durch kombinierte Anwendung mit den Dihalogenacetamiden bei gleichbleibender herbizider Wirkung entscheidend verbessert wird.

Die Versuchsserien wurden im Gewächshaus durchgeführt. Plastikpikierkisten von 51 cm Länge, 32 cm Breite und 6 cm Höhe wurden mit lehmigem Sand von pH 6 bis 7 und etwa 1,5 % bis 3 % Humus gefüllt. In dieses Substrat wurde Mais (Zea mays) und Gerste (Hordeum vulgare) in Reihen flach eingesät. Außerdem wurden Echinochloa crus-galli und Alopecurus myosuroides als unerwünschte Pflanzen breitwürfig hinzugegeben. Der nicht sterilisierte Boden enthielt zusätzlich lebensfähige Unkrautsamen, welche zur Population unerwünschter Pflanzen beitrugen. Dadurch wurde ein mit Kulturpflanzen bestellter und mit Unkräutern verseuchter Acker simuliert.

Wirkstoffe und Antagonisten wurden sowohl einzeln als auch in den beschriebenen Mischungen aufgebracht. Dazu verteilte man sie, emulgiert oder suspendiert in Wasser als Trägermedium, und spritzte sie mittels fein verteilender Düsen unmittelbar nach der Saat und vor Auflaufen der Testpflanzen auf die Erdoberfläche. In einigen Fällen wurden die Mittel auch vor Einsaat der Kulturpflanzen in den Boden eingemischt. Bei der Samenbehandlung puderte man die Samen mit einer festen Formulierung der Wirkstoffe

oder man tränkte die Samen der Kulturpflanzen über einen gewissen Zeitraum in einer flüssigen Aufbereitung (Suspension, Emulsion, Lösungen) der antagonistischen Verbindungen (Samenquellung, seed soaking), trocknete sie so weit, daß die Samen nicht aneinanderklebten und säte sie danach aus. Auch eine Inkrustierung der Samen ist möglich (seed coating). Nach Einsaat der Samen und Behandlung mit dem Herbizid wurden die Kisten beregnet und mit durchsichtigen Plastikhauben abgedeckt, bis die Pflanzen aufgelaufen waren. Durch diese Maßnahmen wurde ein gleichmäßiges Keimen und Anwachsen der Pflanzen ermöglicht. Die Kisten werden in einem Temperaturbereich von durchschnittlich 15 bis 25° C aufgestellt.

Die so angelegten Gewächshausversuche wurden beobachtet, bis der Mais 3 bis 5 Blätter entwickelt hatte. Nach diesem Stadium waren Schädigungen durch die herbiziden Mittel nicht mehr zu erwarten. Bei der Gerste erstreckte sich die Versuchsdauer auf 2 bis 3 Wochen.

Die Wirkung der Mittel wurde nach einer Skala von 0 bis 100 vorgenommen. Hierbei bedeutet 0 normaler Aufgang und Entwicklung der Pflanzen, bezogen auf die unbehandelte Kontrolle. 100 entsprach einem völligen Ausbleiben der Keimung bzw. Absterben der Pflanzen.

Als Vergleichsmittel dienten herbizide Mittel, die neben dem herbiziden Acetanilid das aus der DE-OS 22 18 097 bekannte N-Dichloracetyl-morpholin (V) als antagonistisches Mittel enthielten.

Tabelle 3 - Verbesserung der Verträglichkeit von 2-(Chlor-2',6'-dimethyl-N-(pyrazolyl-1-yl-methyl)-acetanilid (A) für Mais durch Zumischung antagonistischer Verbindungen bei Vorauflaufanwendung im Gewächshaus

| Herbizider Wirkstoff | Antagonistische Verbindung | Aufwandmenge [kg/ha] | Testpflanzen und Schädigung [%] Zea mays | Echinochloa crus-galli |
|---|---|---|---|---|
| A | - | 1,0 | 64 | 100 |
| A | V | 1,0 + 0,125 | 37 | 100 |
| A | 23 | 1,0 + 0,125 | 8 | 100 |
| | | 1,0 + 0,1 | 8 | 100 |
| | | 1,0 + 0,06 | 12 | 100 |
| A | 21 | 1,0 + 0,25 | 12 | 100 |
| | | 1,0 + 0,1 | 18 | 100 |
| A | 1 | 1,0 + 0,125 | 0 | - |

0 = normaler Aufgang, keine Schädigung
100 = Pflanzen nicht aufgelaufen oder abgestorben

Tabelle 4 - Verbesserung der Verträglichkeit von 2-(Chlor-2',6'-dimethyl-N-(pyrazolyl-1-yl-methyl)-acetanilid (A) für Getreide durch eine antagonistische Verbindung bei Vorauflaufanwendung im Gewächshaus

| Herbizider Wirkstoff | Antagonistische Verbindung | Aufwandmenge [kg/ha] | Testpflanzen und Schädigung [%] Hordeum vulgare | Alopecurus myosuroides |
|---|---|---|---|---|
| A | - | 0,5 | 65 | 90 |
| A | 25 | 0,5 + 0,5 | 15 | 95 |

0 = normaler Aufgang, keine Schädigung
100 = Pflanzen nicht aufgelaufen oder abgestorben

Tabelle 5 - Verbesserung der Maisverträglichkeit von 2—Chlor-2',6'-dimethyl-N--(pyrazolyl-1-yl-methyl)-acetanilid (A) durch Saatgutbeizung

| Herbizider Wirkstoff | Antagonistische Verbindung | Dosierung (Wirkstoff + antagonistische Verbindung) | Methode | Testpflanzen und Schädigung [%] Zea mays | Echinochloa crus-galli |
|---|---|---|---|---|---|
| A | - | 1,0 | VAS +) | 68 | 99 |
| A | 23 | 1,0 + 250 g pro 100 kg Saatgut | VAS als Samenbeizung ++ | 0 | 100 |
| A | 23 | 1,0 + 50 g pro 100 kg Saatgut | VAS als Samenbeizung | 0 | 100 |
| A | 22 | 1,0 + 250 g pro 100 kg Saatgut | VAS als Samenbeizung | 10 | 100 |
| A | 22 | 1,0 + 50 g pro 100 kg Saatgut | VAS als Samenbeizung | 10 | 100 |

+VAS = Vorauflaufspritzung nach der Einsaat von Kultur und unerwünschten Pflanzen

++ = von Zea mays

Tabelle 6 - Verbesserung der Maisverträglichkeit von 2-Chlor-2',6'-dimethyl-N--(2-methoxy-ethyl)-acetanilid (B) und von 2-Chlor-2'-methyl-6'-ethyl--N-(ethoxymethyl)-acetanilid (C) für Mais bei Vorauflaufanwendung im Gewächshaus

| Herbizider Wirkstoff | Antagonistische Verbindung | Aufwandmenge [kg/ha] | Testpflanzen und Schädigung [%] Kulturpflanze Zea mays |
|---|---|---|---|
| B | - | 1,0 | 45 |
| B | 23 | 1,0 + 0,25 | 0 |
| | 23 | 1,0 + 0,125 | 5 |
| C | | 2,0 | 32 |
| C | 23 | 2,0 + 0,25 | 5 |

**Patentansprüche**

1. Heterocyclische Dihalogenacetamide der Formel

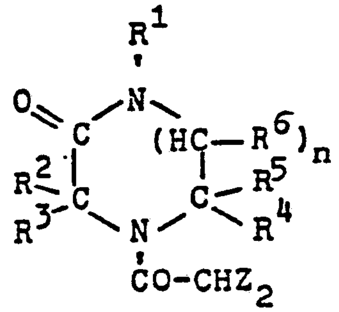

(I),

in der
$R^1$ Wasserstoff oder Alkyl mit 1 bis 4 Kohlenstoffatomen,
$R^2$ Wasserstoff oder Methyl,
$R^3$ Wasserstoff oder Alkyl mit 1 bis 3 Kohlenstoffatomen,
$R^4$ Wasserstoff, Alkyl mit 1 bis 4 Kohlenstoffatomen oder Cyclohexyl,
$R^5$ Wasserstoff oder Methyl,
$R^6$ Wasserstoff oder Methyl,
Z Chlor oder Brom und
n 0 oder 1 bedeuten oder in der $R^2$ zusammen mit $R^3$ einen Pentamethylenrest bildet, oder in der $R^4$ zusammen mit $R^5$ einen Tetra- oder Pentamethylenrest bildet, wenn n 0 ist, oder in der $R^5$ zusammen mit $R^6$ einen Tetramethylenrest bildet, wenn n 1 ist,
mit der Maßgabe, daß $R^1$, $R^2$, $R^3$, $R^4$, $R^5$ und $R^6$ nicht gleichzeitig Wasserstoff bedeuten, wenn n 1 ist.

2. 1-Dichloracetyl-2-isopropyl-4-oxo-imidazolidin.

3. 1-Dichloracetyl-2,2-dimethyl-3-oxo-piperazin.

4. 1-Dichloracetyl-2-methyl-3-oxo-5,6-tetramethylen-piperazin.

5. Verfahren zur Herstellung von heterocyclischen Dihalogenacetamiden der Formel I

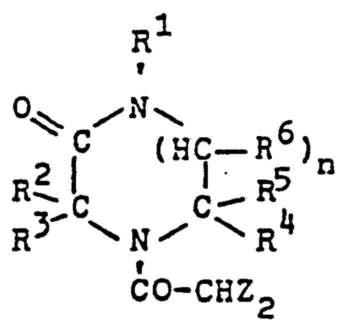

(I),

in der
$R^1$ Wasserstoff oder Alkyl mit 1 bis 4 Kohlenstoffatomen,
$R^2$ Wasserstoff oder Methyl,
$R^3$ Wasserstoff oder Alkyl mit 1 bis 3 Kohlenstoffatomen,
$R^4$ Wasserstoff, Alkyl mit 1 bis 4 Kohlenstoffatomen oder Cyclohexyl,
$R^5$ Wasserstoff oder Methyl,
$R^6$ Wasserstoff oder Methyl,
Z Chlor oder Brom und
n 0 oder 1 bedeuten oder in der $R^2$ zusammen mit $R^3$ einen Pentamethylenrest bildet oder in der $R^4$ zusammen mit $R^5$ einen Tetra- oder Pentamethylenrest bildet, wenn n 0 ist, oder in der $R^5$ zusammen mit $R^6$ einen Tetramethylenrest bildet, wenn n 1 ist,
mit der Maßgabe, daß $R^2$, $R^3$, $R^4$, $R^5$ und $R_6$ nicht gleichzeitig Wasserstoff bedeuten, wenn n 1 ist, dadurch gekennzeichnet, daß man Amine der Formel

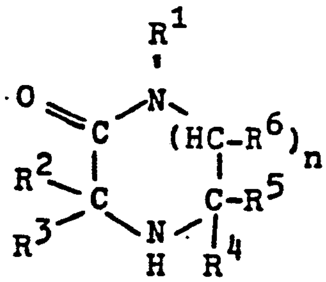

(III),

in der
$R^1$ bis $R^6$ und n die oben genannten Bedeutungen haben,
mit Dihalogenacetylchloriden der Formel $Z_2CH\text{-}COCl$, in der Z Chlor oder Brom bedeutet, in Gegenwart eines halogenwasserstoffbindenden Mittels und eines Lösungsmittels umsetzt.

6. Herbizides Mittel, enthaltend ein Acetanilid der Formel

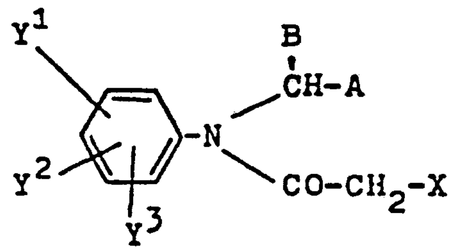

(II),

in der
$Y^1$ Wasserstoff, einen unverzweigten oder verzweigten Alkyl- oder Alkoxyrest mit bis zu 5 Kohlenstoffatomen,
$Y^2$ und $Y^3$ Wasserstoff, Halogen einen unverzweigten oder verzweigten Alkyl- oder Alkoxyrest mit bis zu 5 Kohlenstoffatomen oder
$Y^1$ zusammen mit $Y^2$ eine orthoständig verknüpfte, gegebenenfalls durch unverzweigte oder verzweigte Alkylgruppen mit bis zu 4 Kohlenstoffatomen substituierte Alkylenketten mit bis zu 6 Kohlenstoffatomen,
X Chlor oder Brom,
A einen Alkoxy- oder Alkoxyalkylrest mit bis zu 4 Kohlenstoffatomen oder einen Azolylrest, der einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, wobei A auch für Salze der Azolylreste stehen kann, die 2 oder 3 Stickstoffatomen enthalten, und
B Wasserstoff oder Methyl bedeuten, als herbiziden Wirkstoff und ein heterocyclisches Dihalogenacetamid der Formel

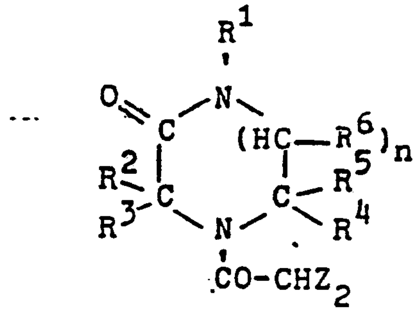

(I),

in der
$R^1$ Wasserstoff oder Alkyl mit 1 bis 4 Kohlenstoffatomen,
$R^2$ Wasserstoff oder Methyl,
$R^3$ Wasserstoff oder Alkyl mit 1 bis 3 Kohlenstoffatomen,
$R^4$ Wasserstoff, Alkyl mit 1 bis 4 Kohlenstoffatomen oder Cyclohexyl,
$R^5$ Wasserstoff oder Methyl,
$R^6$ Wasserstoff oder Methyl,
Z Chlor oder Brom und
n 0 oder 1 bedeuten oder in der $R^2$ zusanmen mit $R^3$ einen Pentamethylenrest bildet, oder in der $R^4$ zusammen mit $R^5$ einen Tetra- oder Pentamethylenrest bildet, wenn n 0 ist, oder in der $R^5$ zusammen mit $R^6$ einen Tetramethylenrest bildet, wenn n 1 ist, mit der Maßgabe, daß $R^1$, $R^2$, $R^3$, $R^4$, $R^5$ und $R^6$ nicht gleichzeitig Wasserstoff bedeuten, wenn n 1 ist.

7. Herbizides Mittel nach Anspruch 6, dadurch gekennzeichnet, daß das Anteilverhältnis Acetanilid: heterocyclischem Dihalogenacetamid bei gemeinsamer oder getrennter Ausbringung 1:2 bis 1:0,01 Gewichtsteile beträgt.

8. Verfahren zur selektiven Bekämpfung von unerwünschtem Pflanzenwuchs, dadurch gekennzeichnet, daß man herbizide Mittel nach Anspruch 6 vor, bei oder nach Aussaat der Kulturpflanzen, vor oder während des Auflaufens der Kulturpflanzen ausbringt.

9. Verfahren zur selektiven Bekämpfung von unerwünschtem Pflanzenwuchs, dadurch gekennzeichnet, daß man Acetanilide der Formel II gemäß Anspruch 6 und Dihalogenacetamide der Formel I gemäß Anspruch 1 vor, bei oder nach der Aussaat der Kulturpflanzen, vor oder während des Auflaufens der Kulturpflanzen gleichzeitig oder nacheinander in beliebiger Reihenfolge ausbringt.

10. Verfahren zur Verhinderung von Schädigungen von Kulturpflanzen durch herbizide Acetanilide der Formel II gemäß Anspruch 6, dadurch gekennzeichnet, daß man das Saatgut der Kulturpflanzen mit einer antagonistisch wirkenden Menge eines heterocyclischen Dihalogenacetamids der Formel I gemäß Anspruch 1 behandelt.

## Claims

1. Heterocyclic dihaloacetamides of the formula

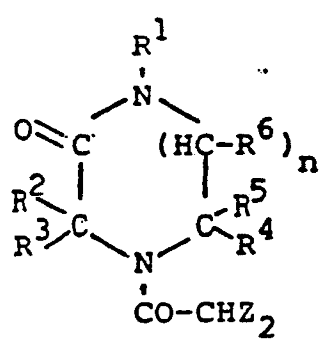

(I),

where
$R^1$ is hydrogen or alkyl of 1 to 4 carbon atoms,
$R^2$ is hydrogen or methyl,
$R^3$ is hydrogen or alkyl of 1 to 3 carbon atoms,
$R^4$ is hydrogen, alkyl of 1 to 4 carbon atoms or cyclohexyl,
$R^5$ is hydrogen or methyl,
$R^6$ is hydrogen or methyl,
Z is chlorine or bromine, and
n is 0 or 1
or where $R^2$ together with $R^3$ is pentamethylene,
or where $R^4$ together with $R^5$ is tetramethylene or pentamethylene if n is 0,
or where $R^5$ together with $R^6$ is tetramethylene if n is 1, with the proviso that $R^1$, $R^2$, $R^3$, $R^4$, $R^5$ and $R^6$ do not simultaneously denote hydrogen if n is 1.

2. 1-Dichloroacetyl-2-isopropyl-4-oxo-imidazolidine.

3. 1-Dichloroacetyl-2,2-dimethyl-3-oxo-piperazine.

4. 1-Dichloroacetyl-2-methyl-3-oxo-5,6-tetramethylene-piperazine.

5. A process for the preparation of heterocyclic dihaloacetamides of the formula I

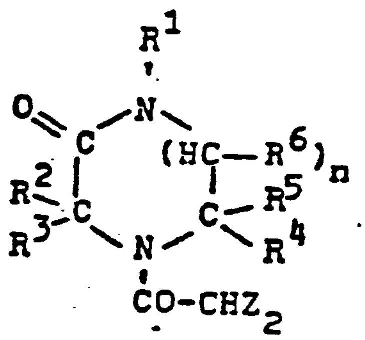

(I),

where
$R^1$ is hydrogen or alkyl of 1 to 4 carbon atoms,
$R^2$ is hydrogen or methyl,
$R^3$ is hydrogen or alkyl of 1 to 3 carbon atoms,
$R^4$ is hydrogen, alkyl of 1 to 4 carbon atoms or cyclohexyl,
$R^5$ is hydrogen or methyl,
$R^6$ is hydrogen or methyl,
Z is chlorine or bromine, and
n is 0 or 1,
or where $R^2$ together with $R^3$ is pentamethylene,
or where $R^4$ together with $R^5$ is tetramethylene or pentamethylene if n is 0,
or where $R^5$ together with $R^6$ is tetramethylene if n is 1, with the proviso that $R^1$, $R^2$, $R^3$, $R^4$, $R^5$ and $R^6$ do not simultaneously denote hydrogen if n is 1,
wherein amines of the formula

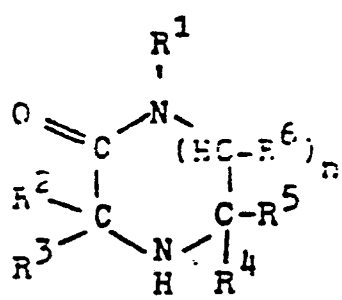

III,

where
$R^1$ to $R^6$ and n have the above meanings,
are reacted with dihaloacetyl chlorides of the formula $Z_2CH\text{-}COCl$.
where Z is chlorine or bromine, in the presence of a hydrogenhalide-binding agent and a solvent.
6. A herbicidal agent containing, as herbicidal active ingredient, an acetanilide of the formula

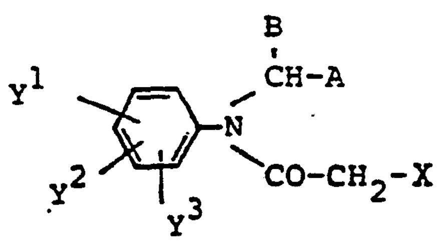

(II),

where
$Y^1$ is hydrogen or straight-chain or branched alkyl or alkoxy of up to 5 carbon atoms, and
$Y^2$ and $Y^3$ are hydrogen, halogen or straight-chain or branched alkyl or alkoxy of up to 5 carbon atoms, or

$Y^1$ and $Y^2$ together are alkylene of up to 6 carbon atoms which is linked in the ortho-position and is unsubstituted or substituted by straight-chain or branched alkyl of up to 4 carbon atoms,

X is chlorine or bromine,

A is alkoxy or alkoxyalkyl of up to 4 carbon atoms, or is azolyl, which may be monosubstituted or polysubstituted by halogen or phenyl, or by alkyl, alkoxy, alkylthio or perfluoroalkyl, each of up to 4 carbon atoms, or by cyano, carboxyl or alkoxycarbonyl where alkoxy is of up to 4 carbon atoms, and A can also be a salt of an azolyl radical, which contains 2 or 3 nitrogen atoms, and

B is hydrogen or methyl,

and a heterocyclic dihaloacetamide of the formula

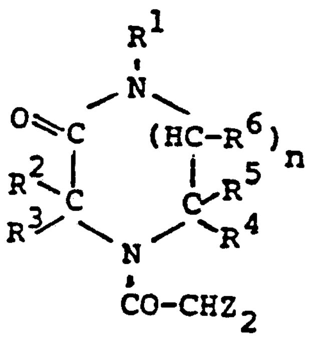

(I),

where

$R^1$ is hydrogen or alkyl of 1 to 4 carbon atoms,

$R^2$ is hydrogen or methyl,

$R^3$ is hydrogen or alkyl of 1 to 3 carbon atoms,

$R^4$ is hydrogen, alkyl of 1 to 4 carbon atoms or cyclohexyl,

$R^5$ is hydrogen or methyl,

$R^6$ is hydrogen or methyl,

Z is chlorine or bromine, and

n is 0 or 1,

or where $R^2$ together with $R^3$ is pentamethylene,

or where $R^4$ together with $R^5$ is tetramethylene or pentamethylene if n is 0,

or where $R^5$ together with $R^6$ is tetramethylene if n is 1, with the proviso that $R^1$, $R^2$, $R^3$, $R^4$, $R^5$ and $R^6$ do not simultaneously denote hydrogen if n is 1.

7. A herbicidal agent as claimed in claim 6, wherein the ratio of acetanilide to heterocyclic dihaloacetamide, applied together or separately, is from 1:2 to 1:0.01 parts by weight.

8. A process for the selective control of unwanted plants, wherein a herbicidal agent as claimed in claim 6 is applied during or after sowing of the crop plants, or before or during emergence of the crop plants.

9. A process for the selective control of unwanted plants, wherein an acetanilide of the formula II as claimed in claim 6 and a dihaloacetamide of the formula I as claimed in claim 1 are applied, either simultaneously or consecutively in any order, before, during or after sowing of the crop plants or before or during emergence of the crop plants.

10. A process for the prevention of damage to crop plants by herbicidal acetanilides of the formula II as claimed in claim 6, wherein the seed of the crop plants is treated with an antagonistically effective amount of a heterocyclic dihaloacetamide of the formula I as claimed in claim 1.

**Revendications**

1.- Dihalogénacétamides hétérocycliques de formule :

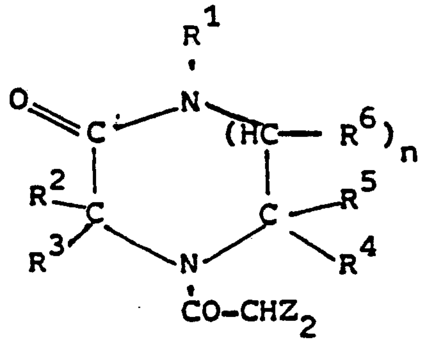

(I)

dans laquelle
$R^1$ représente hydrogène ou alkyle à 1 à 4 atomes de carbone,
$R^2$ hydrogène ou méthyle,
$R^3$ hydrogène ou alkyle à 1 à 3 atomes de carbone
$R^4$ hydrogène, alkyle à 1 à 4 atomes de carbone ou cyclohexyle,
$R^5$ hydrogène ou méthyle
$R^6$ hydrogène ou méthyle
Z chlore ou brome et
n 0 ou 1, ou bien dans laquelle $R^2$ forme avec $R^3$ un reste pentaméthylène, ou bien, dans laquelle $R^4$ forme avec $R^5$ un reste tétra- ou pentaméthylène, quand n est 0, ou bien dans laquelle $R^5$ forme avec $R^6$ un reste tétraméthylène quand n est 1 sous réserve que $R^1$, $R^2$, $R^3$, $R^4$, $R^5$ et $R^6$ ne représentent pas simultanément de l'hydrogène quand n est 1.

2.- 1-dichloracétyl-2-isopropyl-4-oxo-imidazolidine.

3.- 1-dichloracétyl-2,2-diméthyl-3-oxo-pipérazine.

4.-1-dichloracétyl-2-méthyl-3-oxo-5,6-tétraméthylène-pipérazine.

5.-Procédé de préparation de dihalogénacétamides hétérocycliques de formule I

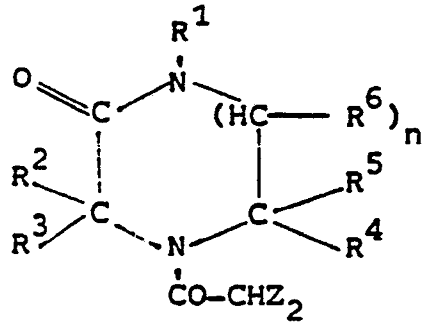

(I)

dans laquelle
$R^1$ represente hydrogène ou alkyle à 1 à 4 atomes de carbone,
$R^2$ hydrogène ou méthyle,
$R^3$ hydrogène ou alkyle à 1 à 3 atomes de carbone,
$R^4$ hydrogène , alkyle à 1 à 4 atomes de carbone, ou cyclohexyle,
$R^5$ hydrogène ou méthyle,
$R^6$ hydrogène ou méthyle,
Z chlore ou brome et
n 0 ou 1, ou bien dans laquelle $R^2$ forme avec $R^3$ un reste pentaméthylène, ou bien, dans laquelle $R^4$ forme avec $R^5$ un reste tétra- ou pentaméthylène, quand n est 0, ou bien dans laquelle $R^5$ forme avec $R^6$ un reste tétraméthylène quand n est 1 sous réserve que $R^1$, $R^2$, $R^3$, $R^4$, $R^5$ et $R^6$ ne représentent pas simultanément de l'hydrogène quand n est 1, caractérisé par le fait que l'on fait réagir des amines de formule

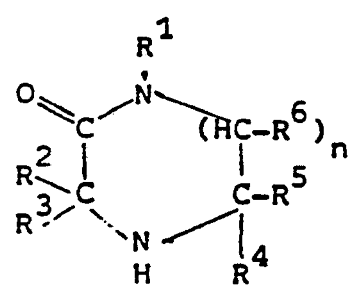

(III)

dans laquelle
$R^1$ à $R^6$ et n ont les significations sus-indiquées avec des dihalogénacétylchlorures de formule $Z_2CH\text{-}COCl$, dans laquelle Z représente chlore ou brome, en présence d'un agent liant les hydracides et d'un solvant.

6.- Agent herbicide, contenant, comme principe herbicide, un acétanilide de formule

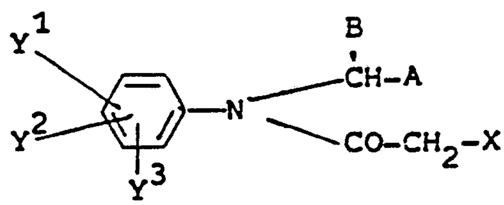

(II)

dans laquelle

$Y^1$ représente hydrogène, un reste alkyle ou alcoxy, ramifié ou non, ayant jusqu'à 5 atomes de carbone,

$Y^2$ et $Y^3$, hydrogène, halogène, un reste alkyle ou alcoxy, ramifié ou non, ayant jusqu'à 5 atomes de carbone, ou

$Y^1$ avec $Y^2$, une chaîne alkylène reliée en position ortho, ayant jusqu'à 6 atomes de carbone, éventuellement substituée par des groupes alkyle, ramifiés ou non, ayant jusqu'à 4 atomes de carbone,

X représente chlore ou brome,

A un reste alcoxy ou alcoxyalkyle ayant jusqu'à 4 atomes de carbone, ou un reste azolyle, qui peut être substitué une fois ou plusieurs fois par halogène, phényle, des restes alkyle, alcoxy, alkylthio ou perfluoralkyle ayant chacun jusqu'à 4 atomes de carbone, cyano, carboxy ou alcoxycarbonyle ayant chacun jusqu'à 4 atomes de carbone dans le groupe alcoxy, A pouvant aussi être mis pour des sels des restes azolyle, qui contiennent 2 ou 3 atomes d'azote, et

B représente hydrogène ou méthyle, et un dihalogénacétamide hétérocyclique de formule

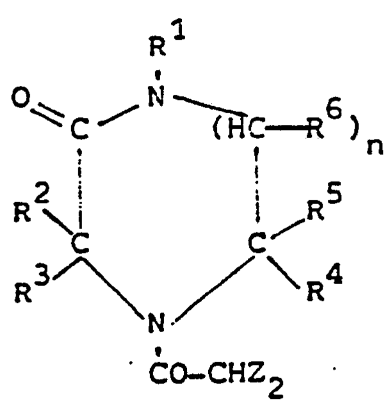

(I)

dans laquelle

$R^1$ représente hydrogène ou alkyle à 1 à 4 atomes de carbone,

$R^2$ hydrogène ou méthyle,

$R^3$ hydrogène ou alkyle à 1 à 3 atomes de carbone,

$R^4$ hydrogène, alkyle à 1 à 4 atomes de carbone ou cyclohexyle,

$R^5$ hydrogène ou méthyle,

$R^6$ hydrogène ou méthyle,

Z chlore ou brome et

n 0 ou 1, ou bien dans laquelle $R^2$ forme avec $R^3$ un reste pentaméthylène, ou bien, dans laquelle $R^4$ forme avec $R^5$ un reste tétra- ou pentaméthylène, quand n est 0, ou bien dans laquelle $R^5$ forme avec $R^6$ un reste tétraméthylène quand n est 1 sous reserve que $R^1$, $R^2$, $R^3$, $R^4$, $R^5$ et $R^6$ ne représentent pas simultanément de l'hydrogène quand n est 1.

7.- Agent herbicide selon la revendication 6 caractérisé par le fait que le rapport entre l'acétanilide et le dihalogénacétamide hétérocyclique, par application simultanée ou séparée , est de 1/2 à 1/0,01 parties en poids.

8.- Procédé de lutte sélective contre une croissance de plantes indésirables, caractérisé par le fait que l'on applique un agent herbicide selon la revendication 6 avant, pendant ou après l'ensemencement des plantes cultivées, avant ou pendant la levée des plantes cultivées.

9.- Procédé de lutte sélective contre une croissance de plantes indésirables, caractérisé par le fait que l'on applique de l'acétanilide de formule II selon la revendication 6 et du dihalogénacétamide de formule I selon la revendication 1, simultanément ou successivement dans un ordre quelconque, avant, pendant ou après l'ensemencement des plantes cultivées, avant ou pendant la levée des plantes cultivées.

10.- Procédé pour empêcher les dommages aux plantes cultivées par l'acétanilide herbicide de formule II selon la revendication 6, caractérisé par le fait que l'on traite la semence des plantes cultivées avec une quantité agissant de façon antagoniste d'un dihalogénacétamide hétérocyclique de formule I selon la revendication 1.